# EUROPEAN PATENT SPECIFICATION

(11) **EP 0 946 170 B1**
(45) Date of publication and mention of the grant of the patent: **12.10.2005**
(21) Application number: 97906922.6
(22) Date of filing: 19.02.1997
(51) Int. Cl.: A61P 3/06, A61P 3/10, A61P 9/10, A61P 29/00, A61K 31/195, A61K 31/19, A61K 31/40, A61K 31/405, A61K 31/54

(54) **DICLOFENAC FOR THE TREATMENT OF VASCULOPATHIES**
DICLOFENAC ZUR BEHANDLUNG VON VASCULOPATHIEN
DICLOFENAC DESTINEE AU TRAITEMENT DES VASCULOPATHIES

(30) Priority: 20.02.1996 US 603147
(43) Date of publication of application: 06.10.1999
(73) Proprietor: EXOCELL, INC., Philadelphia, PA 19104 (US)
(72) Inventor: COHEN, Margo, P., New York, NY 10023 (US); CLEMENTS, Rex, Pineville, PA 18946 (US)
(74) Representative: Patentanwälte Dr. Solf & Zapf
(86) International application number: PCT/US1997/002622
(87) International publication number: WO 1997/029746

(56) References cited:
- US-A- 4 060 626
- US-A- 5 245 046
- M.A.M. VAN BOEKEL ET AL.: "Glycation of human serum albumin: inhibition by Diclofenac" BIOCHIM. BIOPHYS. ACTA, vol. 1120, no. 2, 9 April 1992 (1992-04-09), pages 201-204, XP001011208

## Description

This invention is related to the novel application of [2-[(2,6-dichlorophenyl)amino]benzene acetic acid] which bind to albumin and to low density lipoproteins for the prevention of diabetic angiopathies. The invention further relates to the ability of said compound with albumin-binding properties to inhibit the nonenzymatic glycation of albumin and thereby protect against the formation of glycated albumin which possesses deleterious biologic effects. The invention relates to the ability of said compound with low density lipoprotein-binding properties to inhibit the nonenzymatic glycation of low density lipoproteins, and thereby protect against the formation of glycated low density lipoproteins which promote the formation of cholesterol-laden cells, and enhance the clearance of LDL-cholesterol to lower serum cholesterol levels.

### Background of the Invention

Nonenzymatic glycation is a condensation reaction between glucose and reactive epsilon amino groups of lysine residues in proteins. The reaction is initiated with attachment of the aldehyde function of acylic glucose via nucleophilic addition, forming an aldimine, also known as a Schiff base. This intermediate undergoes an Amadori rearrangement to form an amino-deoxyfructose derivative (fructosyllysine) in stable ketoamine linkage. The Amadori product may give rise to a heterogeneous group of poorly defined advanced glycation end products (AGE), the formation of which is glucose-independent and is believed to evolve through various rearrangement, dehydration, oxidation and polymerization reactions. Prior art has suggested that AGE-modified proteins have a pathophysiologic role in disorders associated with diabetes and aging, and that inhibition of AGE-crosslink formation might be beneficial in such disorders (Brownlee *et al*, *Science* 232:1629, 1986; *New Engl J Med* 318:1315, 1988; Vlassara, *J Lab Clin Med* 124:19, 1994). However, recent experimental work indicates that certain proteins modified by Amadori glucose adducts are important pathogenetic factors in the development of vascular disease, especially that associated with diabetes. Glycated proteins principally exist *in vivo* as Amadori products and their concentration, driven by the ambient glucose concentration to which a protein is subject during its residence time in the circulation, is significantly increased in diabetes with exposure to a hyperglycemic milieu (Cohen and Hud, *J Immunol Meth* 122:279, 1989; Cohen *et al, Eur J Clin Chem Clin Biochem* 31:707, 1993).

Experimental studies have shown that Amadori-modified glycated albumin has distinct biologic effects that unmodified albumin does not possess. These glycated albumin-induced effects, which include stimulation of matrix production in the renal glomerulus and the retinal microvasculature, mimic the changes characteristic of diabetic complications, are likely mediated by ligand-receptor systems specific for the glucose-modified epitope in the glycated protein, and can be prevented by molecules capable of reacting with the Amadori product in glycated albumin (Ziyadeh and Cohen, *Molec Cell Biochem* 125:19, 1993; Cohen *et al, Molec Cell Biochem* 151:61, 1995; Cohen and Ziyadeh, *Kidney Int* 45:475, 1995; Cohen *et al, J Clin Invest* 95:2338, 1995; Wu and Cohen, *Biochem Biophys Res Comm* 207:521, 1995). Such molecules may be monoclonal antibodies or other compounds which specifically bind to the fructosyllysine epitope present on glycated albumin but not present on nonglycated albumin, and which are disclosed in U.S. Patent No. 5,223,392 and U.S. Patent 5,518,720.

Nonenzymatic glycation of apolipoprotein B (apo B), the principal protein of the cholesterol carrying low density lipoproteins (LDL), is a post-secretory modification of LDL that affects its amerogenic potential. Apo B is the protein determinant for the cellular recognition and uptake of LDL by the high affinity LDL receptor. Binding of apo B to LDL receptors results in internalization and degradation of LDL, promoting the clearance of LDL from plasma and regulating intracellular cholesterol handling and biosynthesis. Glycation of LDL diminishes this high affinity LDL receptor-mediated uptake and degradation (Klein *et al, Diabetes* 44:1093, 1995; Gugliucci *et al, Scand J Lab Clin Invest* 53:125, 1993). On the other hand, glycation of lysine residues in apo B promotes internalization by alternative receptors of monocyte-macrophages that give rise to cholesterol-laden foam cells (Klein *et al, Metabolism* 38:1108, 1989; Klein *et al, Diabetes* 44:1093, 1995; Brown & Goldstein, *Nature* 343:508, 1990). Additionally, glycation alters the rate of clearance of LDL *in vivo,* and may interfere with intracellular handling of cholesterol and regulation of its synthesis (Lopez-Virella *et al, Diobetes* 37:550, 1988; Steinbrecher & Witztum, *Diabetes* 33:130, 1984; Lyons *et al, Diabetologia* 30:916, 1987; Klein *et al, Diabetologia* 33:299, 1990). Further, glycation accelerates free radical production which can increase lipid peroxidation and thereby enhance LDL atherogenicity through formation of modified apo B adducts that are recognized and internalized by scavenger receptors (Gillery *et al, Diabete et Metabolism* 14:25, 1988; Mullarkey *et al, Biochem Biophys Res Comm* 183:932, 1990; Steinberg *et al, New Engl J Med* 320:915, 1989). Glycation *per se* also promotes oxidative damage (Hunt *et al, Biochem J* 291:529, 1993).

Prior art has suggested a pathophysiologic role of AGE-modified LDL in the development of atherosclerosis and that inhibition of AGE-crosslink formation might be beneficial in the treatment of atherosclerotic cardiovascular disease (Brownlee *et al Science* 232:1629, 1986; *New Engl J Med* 318:1315, 1988; Vlassara, *J Lab Clin Med* 124:19, 1994; Bucala *et al, Proc Natl Acad Sci* 90:6434, 1993; *Proc Natl Acad Sci* 91:9441, 1994). However, recent experimental work cited above indicates that apo B/LDL modified by Amadori glucose adducts plays an important role in atherogenesis. Further, glycated LDL principally exists *in vivo* as the Amadori product and its concentration is elevated in people with increased risk for atherosclerotic disease (Cohen *et al, Eur J Clin Chem Clin Biochem* 31:707, 1993; Tames *et al, Atherosclerosis* 93:237, 1992).

The deleterious biologic effects of glycated albumin make it desirable to have the means to prevent the attachment of glucose to lysine-amino groups in albumin and thereby lower glycated albumin concentrations. Such means would beneficially influence the development of diabetic complications by mechanisms different from those disclosed in the prior art, which are designed to neutralize the effects of elevated glycated albumin concentrations. One manner by which lowering of glycated albumin concentrations could be accomplished would be with intensive regimens for control of blood glucose levels. The Diabetes Control and Complications Trial (DCCT) showed that near normalization of circulating glycated protein levels with intensive insulin therapy lowers the risk for development of diabetic nephropathy and retinopathy (*New Engl J Med* 329:977, 1993). However, it is widely appreciated that implementation and maintenance of intensive regimens such as those used in the DCCT are difficult, and that the vast majority of diabetic patients remain significantly hyperglycemic with current anti-diabetic therapies. Another means by which lowering of glycated albumin concentrations could be achieved is with compounds that prevent the condensation of glucose with lysine amino groups. Acetylsalicylic acid (aspirin), by virtue of rapid acetylation of epsilon amino lysine groups, can competitively inhibit this reaction (Rao and Cotlier, *Biochem Biophys Res Comm* 151:991, 1988; Rendell *et al, J Lab Clin Med* 107:286, 1986). However, the impact of widespread protein acetylation is unknown. Moreover, the glycation-inhibiting activity of aspirin is relatively weak and potential therapeutic benefits that might be ascribed to this activity are limited by the rapid hydrolysis and short half-life of acetylsalicylic acid in the blood and by side effects anticipated at doses required to inhibit glycation *in vivo* (Costello and Green, *Arth Rheum* 25:550, 1982; Rowland and Riegelmen, *J Pharm Sci* 57:1313, 1968). Other compounds which lack acetyl groups but bind to albumin in a manner that effectively interferes with the condensation of glucose with free lysine amino groups would be more desirable as glycation inhibitors.

Further, prior art discloses that receptor binding and the influence of glycated albumin on endothelial cell biology are both inhibited by the A717 monoclonal antibody, indicating that the fructosyllysine epitope is required for receptor recognition and the induction of biologic effects. These results demonstrate, that increased glycated albumin *in vivo* promotes endothelial damage, and that blocking this influence with anti-glycated albumin antibodies can protect vascular integrity (Cohen et al., Biochem. Phys. Res. Comm. 218: 72, 1996).

In addition, it is also known from prior art the inhibition of albumin glycation *in vitro* by diclofenac (= 2-[(2,6-dichlorophenyl)amino]benzene acetic acid). These results were compared with the inhibition by Aspirin (acetylsalicylic acid) and it is mentioned that it would be interesting to investigate to which extent this affects glycation *in vivo* and further that possibly the use of antiflammatory drugs has an effect on the levels of glycated albumin currently used as parameter for glycemia in diabetes patients (Martinus et al., Biochim. Biophys. Acta 201:1120, 1992).

The deleterious biologic effects of glycated apo B/LDL make it desirable to have the means to prevent the attachment of glucose to apo B lysine-amino groups and thereby lower glycated LDL concentrations. It would also be desirable to reduce plasma LDL cholesterol levels by lowering glycated LDL and thereby promoting LDL cholesterol clearance. Such means would beneficially influence the development of atherosclerosis by mechanisms different from those disclosed in the prior art which relate to agents that lower cholesterol levels by inhibiting gastrointestinal absorption or cholesterol synthesis, or that prevent cross-linked AGE-modified LDL formation. Such means could be achieved with compounds that bind to apo B/LDL in a manner that effectively interferes with the condensation of glucose with free lysine amino groups and thereby inhibit nonenzymatic glycation.

### Summary of the Invention

The present invention provides the use of 2-[(2,6-dichlorophenyl)amino]benzene acetic acid for the manufacture of a medicament for the prevention and the treatment of at least a disease selected from hypercholesterolemia, diabetic vasculopathy, diabetic nephropathy, diabetic retinopathy, atherosclerosis and atherosclerotic cardiovascular disease.

The present invention provides said medicament for preventing the nonenzymatic glycation of albumin.

The present invention also provides said medicament for preventing the formation of biologically active Amadori glucose adducts in the albumin molecule.

Additionally, the present invention provides said medicament for limiting the generation of biologically active glycated albumin epitopes for interaction with their unique cell-associated receptors.

The present invention is achieved with 2-[(2,6-dichlorophenyl)amino]benzene acetic acid capable of binding to albumin in such a way as to inhibit the reaction of glucose with lysine amino groups in the protein.

Another embodiment of the present invention provides said medicament for preventing vasculopathy and the like in diabetes comprising the step of administering to a diabetic patient said medicament capable of preventing the nonenzymatic glycation of albumin *in vivo.*

Another embodiment of the present invention provides said medicament for preventing vascular complications of diabetes comprising the step of administering said medicament capable of preventing the formation of biologically active Amadori glucose adducts in the albumin molecule.

The present invention provides said medicament for preventing the nonenzymatic glycation of apolipoprotein B/LDL.

The present invention also provides said medicament for preventing the formation of biologically active Amadori glucose adducts in the apo B/LDL molecule.

The present invention is achieved with 2-[(2,6-dichlorophenyl)amino]benzene acetic acid capable of binding to apo B/LDL in such a way as to inhibit the reaction of glucose with lysine amino groups in the protein.

The present invention also provides said medicament for preventing atherosclerotic cardiovascular disease.

Another embodiment of the present invention provides said medicament for preventing atherosclerosis comprising the step of administering to a patient said medicament capable of preventing the nonenzymatic glycation of apo B/LDL *in vivo*.

Another embodiment of the present invention provides said medicament for preventing atherosclerosis comprising the step of administering said medicament capable of preventing the formation of biologically active Amadori glucose adducts in the apo B/LDL molecule.

The present invention also provides said medicament for reducing blood cholesterol levels comprising the step of administering to a patient said medicament capable of lowering LDL cholesterol.

The present invention thus relates to use of 2-[(2,6-dichlorophenyl)amino]benzene acetic acid that is reactive with domain(s) in human albumin and that, by binding to these sites in the structure of albumin, protect the protein against nonenzymatic glycation. The sites bound by 2-[(2,6-dichlorophenyl)amino]benzene acetic acid do not entail acetylation of the protein's lysine amino groups. The present invention also relates to use of 2-[(2,6-dichlorophenyl)amino]benzene acetic acid that are reactive with domain(s) in human apo B/LDL and that, by binding to these sites in the structure of apo B/LDL, protect the protein against nonenzymatic glycation.

### Detailed Deseription

It is a finding of the present invention that 2-[(2,6-dichlorophenyl)amino]benzene acetic acid that bind to human albumin protect the protein from nonenzymatic glycation. This protective action reduces the amount of glycated albumin containing Amadori glucose adducts that is formed upon exposure of the protein to glucose *in vitro* and *in vivo*. On the basis of these findings, the use of 2-[(2,6-dichlorophenyl)amino]benzene acetic acid for the manufacture of a medicament is provided for preventing vasculopathy, nephropathy and retinopathy that occur in persons with diabetes. Diabetic persons display increased levels of glycated albumin in their plasma. This increase is pathogenetically involved in the development of microvascular complications in tissues such as the kidney and eye. The present invention lowers the glycated albumin concentration. A decrease in the plasma glycated albumin concentration results in amelioration of diabetes-associated pathologic changes in the kidney and retina, and in prevention of a decline in kidney function. Protection of albumin from nonenzymatic glycation decreases the amount of glycated albumin ligand available for binding to receptors that are present on kidney and other cells and that mediate the biologic effects of glycated albumin, such as stimulation of extracellular matrix production.

It is also a finding of the present invention that 2-[(2,6-dichlorophenyl)amino]benzene acetic acid that bind to human apo B/LDL protect the protein from nonenzymatic glycation. This protective action reduces the amount of glycated apo B/LDL containing Amadori glucose adducts that is formed upon exposure of the protein to glucose *in vitro* and *in vivo*. Reducing glycated LDL promotes clearance of LDL cholesterol and lowers blood cholesterol concentrations. On the basis of this finding, the use of 2-[(2,6-dichlorophenyl)amino]benzene acetic acid for the manufacture of a medicament is provided for preventing atherosclerotic cardiovascular disease. Persons such as those afflicted with diabetes who are at increased risk for atherosclerosis display increased levels of glycated apo B/LDL in their plasma. This increase is pathogenetically involved in the development of atherosclerotic cardiovascular disease. Persons with elevated serum cholesterol levels (who may also have elevated glycated apo B/LDL levels), are at increased risk for atherosclerotic cardiovascular disease. The present invention lowers the glycated apo B/LDL concentration and the serum cholesterol concentration. A decrease in the plasma glycated apo/LDL concentration results in amelioration of LDL cholesterol-associated pathologic changes in the blood vessels and in prevention of atherosclerotic lesions. A decrease in the serum cholesterol concentration lowers the risk for atherosclerotic cardiovascular disease.

The present invention discloses 2-[(2,6-dichlorophenyl)amino]benzene acetic acid that is capable of binding to sites in the tertiary structure of albumin or of apo B. The binding sites of the disclosed compound can involve different parts of the primary structure of the protein and encompass a lysine residue that is a preferential site of nonenzymatic glycation. The compound is 2-[(2,6-dichlorophenyl)amino]benzene-acetic acid.

The usual recommended dose of 2[(2,6-dichlorophenyl)amino] benzeneacetic acid (DCPB) is about 100-200 mg/day in divided doses. For the purposes of the present invention, other doses below and above this range may be employed. DCPB is a weak acid (pKa = 4) that is virtually completely absorbed from the gastrointestinal tract with peak levels of approximately 2 µg/ml achieved 30-150 minutes after dosing. Most of the absorbed DCPB is bound to the circulating albumin and small amounts are bound to lipoproteins (Chan *et al, J Pharm Sci* 76:105, 1987). It is usually given for 10-14 days for inflammatory conditions. For the purposes of this invention, it may be given for many weeks, months or years.

Fluorescent marker displacement analysis indicates that His 146 and Lys 195 residues in albumin are involved in a high affinity binding site for DCPB, and that there is another binding site for the compound, involving a different part of the primary structure of albumin, in which Lys 199 plays a role (Chamouard *et al, Biochem Pharmacol* 34;1695, 1985). Lys 199 represents a fatty acid binding site, suggesting that dissociation of fatty acid from albumin renders this site available for DCPB binding. Lysine 199 also serves as a preferential site for nonenzymatic glycation *in vitro* and *in vivo* (Garlick *et al, J Biol Chem* 258:6142, 1983). The principal site of albumin glycation *in vivo* is lysine 525, which accounts for about 33% of the overall glycation of this protein (Fluckiger, *Monographs in Atherosclerosis* 13:53, 1985; Iberg *et al, J Biol Chem* 261:13542, 1986), and which also may bind DCPB. Lysine 525 modified by Amadori glucose adducts comprises the epitope recognized by glycated albumin cell receptors, and protection by DCPB of glycation of this residue inhibits this ligand-receptor binding.

For prophylaxis and treatment, 2-[(2,6-dichlorophenyl)amino]benzene acetic acid, as described above, can be administered to block glycatable sites in albumin and apo B/LDL. By binding to such sites, the 2-[(2,6-dichlorophenyl)amino]benzene acetic acid can prevent the formation of Amadori glucose adducts in albumin and apo B/LDL, protect against tissue damage which is caused by excess circulating glycated albumin and glycated apo/LDL and by binding of these glycated proteins to cell recognition sites, and promote the clearance of LDL cholesterol from the circulation.

Administration according to the medicament of the present invention is any medicament which achieves a sufficient concentration of said medicament in the circulation or targeted region of the body to be therapeutically useful. Typically such administration will be oral, although parenteral injection may also be used. Typical doses of said medicament will achieve effective concentrations of albumin-binding and of lipoprotein-binding power. Administration according to the medicament of the present invention may comprise said medicament in a composition with a therapeutically acceptable carrier such as saline solution or in tablet formation, including excipients such as calcium phosphate, starch, sucrose, polyethylene glycol, talc and others.

The above disclosure generally describes the present invention. A more complete understanding can be obtained by reference to the following specific examples. The novelty of the invention resides in the treatment to which it is directed, and the following specific examples provide clear indication that the treatment has been tried and tested.

### Example 1. Inhibition of Plasma Albumin Glycation by DCPB

Samples of human plasma (0.5 ml each) were incubated for four days at 25°C with varying concentrations of glucose in the absence or presence of varying concentrations of DCPB. After dialysis to remove unbound reactants, the amount of glucose attached to albumin as Amadori glucose adduct was determined by analysis in an enzyme linked immunosorbent assay (ELISA) which uses monoclonal antibodies known to specifically react with epitopes found on glycated albumin but which are not found on nonglycated albumin. As shown in **Table 1,** DCPB produced a dose-responsive inhibition of the formation of glucose-modified albumin in human plasma.

**Table 1**

| **Sample** | **Glucose** | **DCPB** | **Glycated Albumin** |
|---|---|---|---|
| | *(mM)* | *(mM)* | *(µg*/*ml)* |
| Plasma | 0 | 0 | 660 |
| Plasma | 20 | 0 | 895 |
| Plasma | 20 | 1 | 603 |
| Phsma | 20 | 5 | 550 |
| Plasma | 20 | 10 | 574 |
| Plasma | 40 | 0 | 1182 |
| Plasma | 40 | 1 | 728 |
| Plasma | 40 | 5 | 636 |
| Plasma | 40 | 10 | 496 |

### Example 2. Inhibition of Plasma LDL Glycation by DCPB

Samples of human plasma (0.5 ml each) were incubated for four days at 25°C with varying concentrations of glucose in the absence or presence of varying concentrations of DCPB. After dialysis to remove unbound reactants, the amount of glucose attached to low density lipoprotein as Amadori glucose adduct was determined by analysis in an enzyme linked immunosorbent assay (ELISA) which uses monoclonal antibodies known to specifically react with epitopes found on glycated apo B/LDL but which are not found on nonglycated apo B/LDL. As shown in **Table 2,** DCPB produced a dose-responsive inhibition of the formation of glucose-modified LDL in human plasma.

**Table 2**

| **Sample** | **Glucose** | **DCPB** | **Glycated apo/LDL** |
|---|---|---|---|
| | *(mM)* | *(µM)* | *(µg*/*ml)* |
| Plasma | 5 | 0 | 11.16 |
| Plasma | 40 | 0 | 15.13 |
| Plasma | 40 | 5 | 11.55 |
| Plasma | 40 | 10 | 9.25 |
| Plasma | 40 | 20 | 7.71 |
| Plasma | 40 | 50 | 7.70 |

### Example 3. DCPB Lowers Glycated Albumin in Diabetic Persons

Blood was obtained from persons with diabetes at the initiation (day 0) and termination (day 14) of 14 days of treatment with DCPB, 200 mg/day orally in divided doses. The plasma concentration of glycated albumin in these samples was determined by enzyme linked immunoassay using monoclonal antibodies known to specifically react with albumin containing Amadori glucose adducts, to be unreactive with unglycated albumin, and to have highest reactivity with the fructosyllysine epitope at lysine 525 of albumin. As shown in **Table 3,** administration of DCPB produced a 30-40% reduction in the amount of circulating glycated albumin.

**Table 3**

| **Patient** | **Glycated Albumin** | **Glycated Albumin (% of total Albumin)** |
|---|---|---|
| | ***(µg*****/*****ml)*** | |
| *Subject 1* | | |
| Day 0 | 1258 | 3.00 |
| Day 14 | 690 | 1.69 |

| *Subject 2* | | |
|---|---|---|
| Day 0 | 508 | 1.38 |
| Day 14 | 296 | 0.80 |

| *Subject 3* | | |
|---|---|---|
| Day 0 | 840 | 2.7 |
| Day 14 | 588 | 1.9 |

### Example 4. In Vivo Protection of LDL Glycation by DCPB

Blood was obtained from human subjects at the initiation (day 0) and termination (day 14) of 14 days of treatment with DCPB, 200 mg/day orally in divided doses. The plasma concentration of glycated LDL in these samples was determined by enzyme linked immunoassay using monoclonal antibodies known to specifically react with LDL containing Amadori glucose adducts, and to be unreactive with nonglycated LDL or other plasma proteins. As shown in **Table 4,** administration of DCPB produced a 10-20% reduction in the amount of circulating glycated LDL.

**Table 4**

| | **µg/ml Glycated apo/LDL** |
|---|---|
| *Subject 1* | |
| Day 0 | 14.03 |
| Day 14 | 12.81 |

| *Subject 2* | |
|---|---|
| Day 0 | 12.65 |
| Day 14 | 10.71 |

| *Subject 3* | |
|---|---|
| Day 0 | 24.87 |
| Day 14 | 19.30 |

### Example 5. DCPB Lowers Serum Cholesterol

Serum samples were obtained from human subjects at the initiation (day 0) and termination (day 14) of 14 days of treatment with DCPB, 200 mg/day orally in divided doses. Cholesterol concentrations were measured by the cholesterol esterase/cholesterol oxidase method (Sigma Chem Co., St. Louis, MO). As shown in **Table 5,** administration of DCPB produced a 17% reduction in the serum cholesterol concentration

**Table 5**

| | **Cholesterol (mg/dL)** |
|---|---|
| *Subject 1* | |
| Day 0 | 242 |
| Day 14 | 199 |

| *Subject 2* | |
|---|---|
| Day 0 | 210 |
| Day 14 | 175 |

### Example 6. Relative Efficacy of Compounds of Different Chemical Classes in Inhibiting Plasma Albumin Glycation

Samples of human plasma (0.5 ml each) were incubated for four days at 25°C with 40 mM glucose in the presence of compounds from different chemical classes at varying concentrations. After dialysis to remove unbound reactants, the amount of glucose attached to albumin as the Amadori glucose adduct was determined by analysis in ELISA as described in **Example 1.** As shown in **Table 6,** the concentrations at which these compounds produced a 50% inhibition (^{IC}50) in glycated albumin were orders of magnitude greater than that of DCPB.

**Table 6**

| **Compound** | **Class** | **IC**_{**50**} **(µM) for Albumin Glycation** |
|---|---|---|
| 2[(2,6-dichlorophenyl)amino]benzeneacetic acid | Heteroaryl acetic acid | 18 |
| 1-methyl-5-[p-toluoyl]pyrrole-2-acetic acid | Heteroaryl acetic acid | 11.5 x 10⁴ |
| 4-hydroxy-2-methyl-3-[pyrid-2-yl-carbamoyl}-2H-1,2-benzathiazine 1,1-dioxide | Enolic acid | > 10⁶ |
| 2-[(2,6-dichloro-3-methylphenyl)-amino]benzoic acid | Anthranilic acid | > 10⁶ |
| α-methyl-4-[2-methylpropyl] benzeneacetic acid | Aryl propionic acid | 7.8 x 10⁴ |
| 1-[p-chlorobeozoyl]-5-methoxy-2-methylindole-3-acetic acid | Indole acetic acid | 2.7 x 10⁴ |
| [Z]-5-fluoro-2-methyl-1 [p-(methylsulfinyl)phenyl]methylene]-1H-indene-3-acetic acid | Indole acetic acid | 2.2 x 10⁴ |

### Example 7. Relative Efficacy of Compounds of Different Chemical Classes in Inhibiting Plasma LDL Glycation

Samples of human plasma (0.5 ml each) were incubated for four days at 25°C with 40 mM glucose in the presence of compounds from different chemical classes at varying concentrations. After dialysis to remove unbound reactants, the amount of glucose attached to low density lipoprotein as Amadori glucose adduct was determined by analysis in ELISA as described in **Example 2,** As shown in **Table 7,** the concentrations at which these compounds produced an 8-15% reduction in glycated apo B/LDL formation were 1,000 fold greater than that of DCPB.

**Table 7**

| **Compound** | **Concentration Required to Achieve 1-15% Reduction in Apo B/LDL Glycation** |
|---|---|
| Dichlorophenylamino-benzeneacetic acid | 1 x 10⁻⁶M |
| Methyl-toluoyl-pyrrole acetic acid | 1 x 10⁻³M |
| Methyl-methylpropyl-benzeneacetic acid | 1 x 10⁻³M |
| Chlorobenzoyl-methoxy-methyl-indoleacetic | 1 x 10⁻³M |

### Example 8. Inhibition of Albumin Glycation Prevents Ligand Binding to Cell Receptors Specific for Fructosyllysine-Modified Albumin

Renal glomerular mesangial cells were incubated for two hours in buffer containing 10 mM HEPES, pH 7.4, 137 mM NaCl, 11 mM glucose, 4 mM KCl and 5 mM CaCl₂ and 20 µg of fluorescein-conjugated (FITC) glycated albumin (Wu and Cohen, *Biochem Biophys Res Comm* 207:521, 1995). Specific binding of FITC-glycated albumin to mesangial cell receptors, measured by fluroescence with excitation at 485 nm and emission at 530 nm, was determined as the difference between binding in the absence (total binding) and presence (nonspecific binding) of excess unconjugated glycated albumin. The effect of plasma albumin previously incubated with glucose, in the absence or presence of DCPB, on specific binding of FITC-glycated albumin was determined. Albumin glycated by exposure to 40 mM glucose for 4 days effectively competed with FITC-glycated albumin for specific binding to mesangial cell receptors, whereas albumin exposed to 40 mM glucose plus 10 mM DCPB for 4 days did not compete for this specific binding.

### Example 9. Lowering Glycated Albumin Ameliorates Diabetic Nephropathy

Diabetic animals in which plasma glycated albumin concentrations were approximately twice those measured in nondiabetic controls were treated for 8 weeks with a regimen that reduced glycated albumin concentrations to normal but did not affect blood glucose concentrations. Urine and blood samples were obtained at the initiation and termination of therapy for examination of renal function, assessed by serum creatinine concentrations and creatinine clearances. As shown in **Table 8,** reducing glycated albumin in diabetic animals prevented the decline in renal function that was observed in untreated diabetic animals.

**Table 8**

| **Animal** | **Glycated Albumin** | **Serum Creatinine Clearance-** | **Creatinine** |
|---|---|---|---|
| | *(µg*/*ml)* | *(mg*/*dL)* | *(ml*/*hr)* |
| *Nondiabetic* | | | |
| Initiation | 280 | .48 | 4.0 |
| Termination | 285 | .60 | 4.5 |
| | | | |

| *Diabetic - Untreated* | | | |
|---|---|---|---|
| Initiation | 410 | .50 | 6.0 |
| Termination | 415 | .95 | 2.2 |
| | | | |

| *Diabetic - Treated* | | | |
|---|---|---|---|
| Initiation | 420 | .50 | 6.0 |
| Termination | 295 | .62 | 4.1 |

## Claims

1. Use of 2-[(2,6-dichlorophenyl)amino]benzene acetic acid for the manufacture of a medicament for the prevention and the treatment of at least a disease selected from hypercholesterolemia, diabetic vasculopathy, diabetic nephropathy, diabetic retinopathy, atherosclerosis and atherosclerotic cardiovascular disease.

2. Use according to claim 1 wherein the medicament inhibits the formation of Amadori glucose adducts in the albumin molecule or the apolipoprotein B molecule.

3. Use according to claim 2 wherein the medicament reduces fructosyllysine modification of the albumin molecule that occurs at one or more residues selected from the group consisting of lysine 199, lysine 28 1, lysine 439 and lysine 525 and wherein the medicament reduces fructosyllysine modification of the apolipoprotein B protein at one or more lysine residues.

4. Use according to claim 1 wherein the medicament inhibits the formation of biologically active epitopes in glycated albumin or in glycated apolipoprotein B that are recognized by cell associated receptors.

5. Use according to claims 1 to 4 wherein the medicament is formulated for the presentation of doses of about 100-200 mg/day for many weeks, months or years.

6. Use according to claims 1 to 5 wherein the medicament is formulated for oral or parenteral administration.

## Patentansprüche

1. Verwendung von 2-[(2,6-Dichlorphenyl)amino]benzolessigsäure zur Herstellung eines Arzneimittels für die Prävention und die Behandlung von mindestens einer Krankheit, ausgewählt aus Hypercholesterinämie, diabetischer Vaskulopathie, diabetischer Nephropathie, diabetischer Retinopathie, Arteriosklerose und arteriosklerotischer Herzkreislauferkrankung.

2. Verwendung gemäß Anspruch 1, wobei das Arzneimittel die Bildung von Amadori-Glucoseaddukten im Albuminmolekül oder dem Apolipoprotein B-Molekül hemmt.

3. Verwendung gemäß Anspruch 2, wobei das Arzneimittel eine Fructosyllysin-Modifikation des Albuminmoleküls vermindert, die an einem oder mehreren Resten, ausgewählt aus der Gruppe bestehend aus Lysin 199, Lysin 281, Lysin 439 und Lysin 525 ist, auftritt und wobei das Arzneimittel eine Fructosyllysin-Modifikation des Apolipoprotein B-Proteins an einem oder mehreren Lysin-Resten vermindert.

4. Verwendung gemäß Anspruch 1, wobei das Arzneimittel die Bildung von biologisch aktiven Epitopen im glycosylierten Albumin oder im glycosylierten Apolipoprotein B hemmt, die durch zell-assoziierte Rezeptoren erkannt werden.

5. Verwendung gemäß der Ansprüche 1 bis 4, wobei das Arzneimittel für die Darreichung einer Dosis von ungefähr 100 bis 200 mg/Tag über viele Wochen, Monate oder Jahre formuliert ist.

6. Verwendung gemäß der Ansprüche 1 bis 5, wobei das Arzneimittel für eine orale oder parenterale Verabreichung formuliert ist.

## Revendications

1. Utilisation d'acide 2-[-2,6-dichlorophényl)amino]benzène acétique pour la fabrication d'un médicament pour la prévention et le traitement d'au moins une maladie choisie parmi l'hypercholestérolémie, la vasculopathie diabétique, la néphropathie diabétique, la rétinopathie diabétique, l'athérosclérose et les maladies cardiovasculaires athérosclérotiques.

2. Utilisation selon la revendication 1 dans laquelle le médicament inhibe la formation de produits d'addition du glucose d'Amadori dans la molécule d'albumine et la molécule d'apolipoprotéine B.

3. Utilisation selon la revendication 2 dans laquelle le médicament réduit la modification par la fructosyle lysine de la molécule d'albumine qui a lieu au niveau d'un ou plusieurs résidus choisis dans le groupe comprenant la lysine 199, la lysine 281, la lysine 439 et la lysine 525 et dans laquelle le médicament réduit la modification par la fructosyle lysine de la protéine apolipoprotéine B au niveau d'un ou de plusieurs résidus lysine.

4. Utilisation selon la revendication 1 dans laquelle le médicament inhibe la formation d'épitopes actifs sur le plan biologique dans l'albumine glyquée ou dans l'apolipoprotéine B glyquée qui sont reconnus par des récepteurs associés aux cellules.

5. Utilisation selon les revendications 1 à 4 dans laquelle le médicament est formulé pour la présentation de doses d'environ 100 à 200 mg/jour pendant de nombreuses semaines, de nombreux mois ou de nombreuses années.

6. Utilisation selon les revendications 1 à 5 dans laquelle le médicament est formulé pour une administration orale ou parentérale.
